(19) Europäisches Patentamt
European Patent Office
Office européen des brevets

(11) Numéro de publication: **0 073 711**
**B1**

# (12) FASCICULE DE BREVET EUROPEEN

(45) Date de publication du fascicule du brevet: **20.03.85**

(51) Int. Cl.⁴: **C 07 C 59/347**, C 07 C 51/41, A 61 K 31/19

(21) Numéro de dépôt: **82401560.6**

(22) Date de dépôt: **20.08.82**

## (54) Sels de l'acide 2-oxo-1,5-pentane dioique, leur procédé de fabrication et médicaments renfermant ces sels.

(30) Priorité: **26.08.81 FR 8116284**

(43) Date de publication de la demande: **09.03.83 Bulletin 83/10**

(45) Mention de la délivrance du brevet: **20.03.85 Bulletin 85/12**

(84) Etats contractants désignés: **AT BE CH DE GB IT LI LU NL SE**

(56) Documents cités:
**FR - M - 827**
**FR - M - 6 969**
**US - A - 2 841 616**

(73) Titulaire: **S.A. DAUTREVILLE ET LEBAS (Société Anonyme), 4 rue Treilhard, F-75008 Paris (FR)**
Titulaire: **REXIM S.A. Société Anonyme, 3 rue Dagcrno, F-75012 Paris (FR)**

(72) Inventeur: **Richet, Gérard, 88 avenue Robert Schumann, F-02100 Saint-Quentin (FR)**
Inventeur: **Cholet, René, Le Parc de Petit Bourg Allée 24, F-91000 Evry (FR)**

(74) Mandataire: **Bouju, André et al, 38 Avenue de la Grande Armée, F-75017 Paris (FR)**

EP 0 073 711 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne deux nouveaux sels de l'acide 2-oxo-1,5-pentanedioïque.

L'invention vise également le procédé de fabrication de ces sels ainsi qu'un médicament renfermant l'un de ces sels, en tant que substance active.

Le document FR-M-827 décrit l'acide α-céto-glutarique et ses dérivés, en particulier son sel de calcium. Dans ce sel, le calcium est fixé aux deux fonctions acides de l'acide α-cétoglutarique, c'est-à-dire à raison d'un atome de calcium par molécule d'acide. Le médicament renfermant un tel sel en tant que substance active est particulièrement indiqué pour le traitement des troubles du métabolisme nerveux du cerveau. La préparation de tels sels est également décrite dans le brevet US-A No 2841616.

Le brevet FR-M No 6969 décrit des médicaments psychotropes renfermant en tant que substance active les sels de lithium de différents acides tels que l'acide α-cétoglutarique.

Le but de la présente invention est de fournir deux nouveaux sels de l'acide 2-oxo-1,5-pentanedioïque (ou acide α-cétoglutarique) présentant des propriétés thérapeutiques améliorées par rapport à celles des sels connus précités.

Suivant l'invention, le premier sel est constitué par le sel de calcium acide de l'acide 2-oxo-1,5-pentanedioïque qui a pour formule:

$$(COOH-CH_2-CH_2-\underset{\underset{O}{\|}}{C}-COO)_2-Ca^{2+}, x\,H_2O$$

formule dans laquelle x représente un nombre entier compris entre 0 et 4. Cette formule montre que l'une des deux fonctions acides est libre.

La formule brute de ce sel, à l'état anhydre, est:

$$C_{10}H_{10}O_{10}Ca,$$

ce qui correspond à une masse moléculaire égale à 330. Le calcium représente 12,12%, le carbone 36,36%, l'oxygène 48,48% et l'hydrogène 3,03% du poids total.

La solubilité de ce sel à l'état trihydraté, dans l'eau à 20° C, est de 9% en poids.

Le pH d'une solution aqueuse saturée de ce sel est compris entre 3,1 et 3,5.

Suivant l'invention, le second sel est constitué par le sel de lithium acide de l'acide 2-oxo-1,5-pentanedioïque qui a pour formule:

$$COOH-CH_2-CH_2-\underset{\underset{O}{\|}}{C}-COO^-\,Li^+, H_2O$$

La formule brute de ce sel, à l'état anhydre, est:

$$C_5C_5O_5Li,$$

ce qui correspond à une masse moléculaire égale à 152. Le lithium représente 4,60%, le carbone 39,47%, l'oxygène 52,63% et l'hydrogène 3,29% du poids total.

La solubilité du sel de lithium, dans l'eau à 20° C, est de 5,5% en poids.

Suivant le procédé conforme à l'invention, pour fabriquer le sel de l'acide 2-oxo-1,5-pentanedioïque, on fixe des ions calcium ou lithium sur une résine échangeuse d'ions à l'aide d'une solution d'un sel de calcium ou de lithium, puis on fait passer sur cette résine une solution aqueuse d'acide 2-oxo-1,5-pentanedioïque, on recueille l'effluent que l'on concentre jusqu'à cristallisation puis on filtre, lave et sèche les cristaux obtenus.

L'acide 2-oxo-1,5-pentanedioïque de départ peut, par exemple, être préparé de façon connue, par condensation de l'éthyloxalate avec l'éthyl-succinate, en présence d'oxyde de sodium ou de potassium pour obtenir l'éthyloxalylsuccinate, puis par hydrolyse de cet éthyloxalylsuccinate avec l'acide chlorhydrique, selon la suite de réactions suivantes:

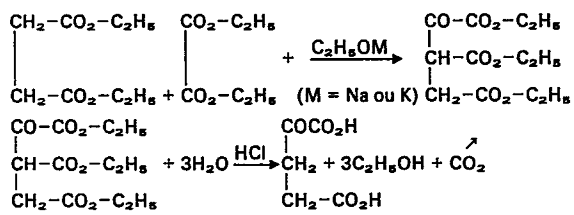


Selon une version préférée du procédé conforme à l'invention, on utilise, en tant que résine échangeuse d'ions, une résine faiblement acide, et en particulier une résine de type carboxylique.

Selon une version avantageuse du procédé, on fixe les ions calcium sur la résine par percolation d'une solution d'hydroxyde de calcium ou de lithium saturée ou d'une solution d'acétate de calcium ou de lithium.

Selon une variante de l'invention, il est également possible de fixer des ions calcium sur la résine par percolation d'une solution de chlorure de calcium après avoir fixé sur cette résine des ions de sodium ou d'ammonium.

Selon une autre caractéristique de l'invention, on concentre l'effluent aqueux de la résine sous une pression réduite.

Pour obtenir des résultats particulièrement satisfaisants, on fait passer sur la résine une solution aqueuse sensiblement normale d'acide 2-oxo-1,5-pentanedioïque, puis de l'eau déminéralisée jusqu'à l'obtention d'un effluent neutre.

On donne ci-après, à titre d'exemple non limitatif, un mode opératoire détaillé de la fabrication du sel de calcium acide de l'acide 2-oxo-1,5-pentanedioïque.

On a chargé 1 l de résine échangeuse d'ions faiblement acide du type carboxylique par percolation d'une solution aqueuse d'acétate de calcium à 10%. On a ainsi fixé 33 g de calcium sur cette résine. On a alors fait passer par percolation dans cette résine une solution aqueuse d'acide 2-oxo-1,5-pentanedioïque.

Pour préparer cette solution, on dissout 260 g d'acide 2-oxo-1,5-pentanedioïque dans 3,562 l d'eau pour obtenir une solution normale d'acide que l'on a fait passer par percolation dans la résine échangeuse d'ions. On a ensuite fait passer dans cette résine de l'eau déminéralisée jusqu'à l'obtention d'un effluent neutre.

La totalité de l'effluent a alors été concentrée sous pression réduite jusqu'à un volume de 400 ml que l'on a laissé cristalliser pendant 24 h à 15° C.

Les cristaux formés ont alors été essorés puis lavés à l'eau et séchés sous pression réduite à 60° C.

On a ainsi obtenu 265 g de sel acide de calcium trihydraté contenant 10,40% de calcium (théorie 10,42%).

La solution mère et la solution de lavage ont ensuite été réunies puis concentrées sous pression réduite jusqu'à un volume de 140 ml, puis laissées au repos pendant 24 h à 15° C en amorçant la cristallisation.

On a ainsi isolé un deuxième jet de cristallisation pesant 23 g après séchage et contenant 10,38% de calcium, ce qui porte le rendement du procédé à 89%.

Pour préparer le sel acide de lithium de l'acide 2-oxo-1,5-pentanedioïque on procède comme suit:

Par percolation d'une solution d'acétate de lithium à 11% sur 1 l de résine échangeuse d'ions faiblement acide, 16,45 g de lithium sont fixés. On passe ensuite une solution normale d'acide 2-oxo-1,5-pentanedioïque (soit 350 g dissous dans 4,8 l $H_2O$), puis de l'eau désionisée jusqu'à obtention d'un effluent neutre.

Tout l'effluent acide est concentré sous pression réduite jusqu'à bonne cristallisation du sel, soit un volume de 450 ml. Après refroidissement à température ambiante, les cristaux sont essorés, lavés avec de l'éthanol, séchés sous pression réduite à 60° C. 359 g de sel acide de lithium monohydraté contenant 4,20% de lithium ont été obtenus, soit avec un rendement de 92%.

L'invention vise également un médicament pour le traitement d'affections du système nerveux central, renfermant en tant que substance active le sel de calcium ou de lithium acide conforme à l'invention.

On indique ci-après les résultats des essais toxicologiques et pharmacologiques qui ont été réalisés sur le sel de calcium acide de l'acide 2-oxo-1,5-pentanedioïque.

*Toxicité:*

On a vérifié que les doses létales 50 (DL50) du sel acide de calcium de l'acide 2-oxo-1,5-pentanedioïque sont, chez la souris, de 10 g/kg dans le cas d'une administration orale et de 2 g/kg dans le cas d'une administration intrapéritonéale.

Un groupe de rats a été soumis à un traitement quotidien par le sel acide de calcium de l'acide 2-oxo-1,5-pentanedioïque pendant une durée de 30 d. Aucune anomalie dans les constantes biologiques, hématologiques et les examens histologiques de différents viscères n'a été relevée pour des doses allant jusqu'à 1 g/kg/d.

*Activité du sel de calcium de l'acide 2-oxo-1,5-pentanedioïque sur le système nerveux central de la souris*

1. *Réduction du ptosis réserpinique*

Chez la souris, à la dose de 0,5 g/kg à 1 g/kg de sel acide de calcium administré *per os*, le ptosis réserpinique est réduit de façon significative 1 h après l'administration du sel.

2. *Antagonisme de l'action myorelaxante de la chlorpromazine (CPZ)*

La chlorpromazine induit une myorelaxation chez la souris. On constate une réduction de 37,5% pour une dose de 1 g/kg et une réduction de 62,5% pour une dose de 2 g/kg.

3. *Test du rotarod* (cylindre tournant)

Dans ce test, la souris tourne sur un cylindre animé d'un mouvement rotatif, perd l'équilibre et tombe après administration de chlorpromazine (CPZ). On constate un antagonisme du CPZ chez la souris dès la dose de 0,5 g/kg. On a obtenu également une réduction du déséquilibre moteur induit par le CPZ.

4. *Réduction de la narcose barbiturique*

Les résultats de ce test se sont révélés positifs chez la souris.

5. *Réduction de l'hypothermie réserpinique*

Ce test a fourni des résultats positifs après administration à la souris de doses de sel acide de calcium comprises entre 0,5 et 1 g/kg *per os.*

6. *Test de la planche à trous*

Sur une planche percée de trous, la souris, parcourant la surface, explore les divers trous rencontrés.

Après un seul traitement au moyen du sel conforme à l'invention, on constate une inhibition de la diminution d'exploration obtenue par le méprobamate.

Après 3 d de prétraitement, on constate une augmentation notable de l'activité exploratrice par rapport à des animaux témoins.

7. *Activité motrice*

Ce test a montré que l'administration à des souris du sel, conforme à l'invention, provoque une augmentation de leur activité motrice.

8. *Catalepsie*

Ce test a montré que le sel conforme à l'invention s'oppose à l'activité catatonique de la prochlorpémazine.

*Etude pharmacocinétique*

Dans cette étude, on a administré à des rats le sel conforme à l'invention dont la molécule a été marquée au $C^{14}$ radioactif. Cette étude a montré le passage de la molécule à travers la barrière hémato-encéphalique et la conversion de celle-ci en acide γ-aminobutyrique (GABA) au niveau cérébral, le principal métabolite d'élimination étant l'acide glutamique.

*Essais cliniques:*

1. *Effets correctifs du sel acide de calcium de l'acide 2-oxo-1,5-pentanedioïque sur l'hypoxie*

Le test est réalisé sur le patient après le passage de celui-ci en caisson sous une hypoxie à 12,5% d'$O_2$.

On a constaté:

– à la dose aiguë de 12 g *per os*, le sel de calcium apparaît comme un activateur efficace capable de contrebalancer l'effet d'une hypoxie à 12,5% d'$O_2$;

– à la dose de 4 g/d *per os* durant 8 d, les effets obtenus sont plus nets sur les tracés des électrocardiogrammes et encéphalogrammes, sur le comportement et la thymie que ceux constatés avec le piracétam.

2. *Correction des troubles de la mémoire, des états névrotiques (anxiophobiques), et des états psychasténiques*

Une étude, effectuée sur soixante patients auxquels ont été administrés quotidiennement en deux prises 4 g de sel conforme à l'invention pour les uns et du placebo pour les autres, a permis de montrer l'excellente efficacité du sel de calcium acide à l'égard des affections précitées.

Ces essais montrent que le sel de calcium acide de l'acide 2-oxo-1,5-pentanedioïque présente chez l'homme d'intéressantes propriétés psychoanaleptiques.

Les essais effectués sur le sel de lithium acide de l'acide 2-oxo-1,5-pentanedioïque ont montré que ce sel présentait des propriétés pharmacologiques et thérapeutiques comparables à celles du sel de calcium.

La posologie du médicament conforme à l'invention, renfermant en tant que substance active le sel de calcium ou de lithium, est la suivante:

4 g/d de substance active, pour les adultes;

2 g/d de substance active, pour les enfants.

Le médicament conforme à l'invention peut être présenté sous forme pharmaceutique administrable par voie orale, telle que des cachets ou comprimés effervescents renfermant entre 0,5 et 2 g de substance active.

Le médicament conforme à l'invention peut également être présenté sous forme d'ampoules renfermant une solution injectable par voie intrapéritonéale, dosée par exemple à 1 g de substance active.

**Revendications** pour les Etats contractants BE, CH, DE, GB, IT, LI, LU, NL, SE

1. Sel de calcium de l'acide 2-oxo-1,5-pentanedioïque, caractérisé en ce qu'il a pour formule:

$$(COOH-CH_2-CH_2-\underset{\underset{O}{\|}}{C}-COO)_2^{-}Ca^{2+}, x\ H_2O$$

dans laquelle x est un nombre entier compris entre 0 et 4.

2. Sel de lithium de l'acide 2-oxo-1,5-pentanedioïque, caractérisé en ce qu'il a pour formule:

$$COOH-CH_2-CH_2-\underset{\underset{O}{\|}}{C}-COO^-\ Li^+, H_2O$$

3. Procédé de fabrication d'un sel acide conforme à l'une des revendications 1 ou 2, caractérisé en ce que l'on fixe des ions de calcium ou de lithium sur une résine échangeuse d'ions à l'aide d'une solution d'un sel de calcium ou de lithium, puis on fait passer sur cette résine une solution aqueuse d'acide 2-oxo-1,5-pentanedioïque, on recueille l'effluent que l'on concentre jusqu'à cristallisation puis on filtre, lave et sèche les cristaux obtenus.

4. Procédé selon la revendication 3, caractérisé en ce que la résine échangeuse d'ions est une résine faiblement acide.

5. Procédé selon l'une des revendications 3 ou 4, caractérisé en ce que l'on fixe des ions de calcium ou de lithium sur la résine par percolation d'une solution d'hydroxyde, ou d'acétate de calcium ou de lithium.

6. Procédé selon l'une des revendications 3 ou 4, caractérisé en ce que l'on fixe des ions de calcium sur la résine par percolation d'une solution de chlorure de calcium après avoir fixé sur cette résine des ions de sodium ou d'ammonium.

7. Procédé selon l'une des revendications 3 à 6, caractérisé en ce que la résine échangeuse d'ions est une résine de type carboxylique.

8. Procédé selon l'une des revendications 3 à 7, caractérisé en ce que l'on concentre l'effluent aqueux de la résine sous pression réduite.

9. Procédé selon l'une des revendication 3 à 8, caractérisé en ce que l'on fait passer sur la résine une solution aqueuse, sensiblement normale, d'acide 2-oxo-1,5-pentanedioïque puis de l'eau déminéralisée jusqu'à l'obtention d'un effluent neutre.

10. Médicament pour le traitement des affections du système nerveux central, caractérisé en ce qu'il renferme en tant que substance active un sel conforme à l'une des revendications 1 ou 2.

11. Médicament conforme à la revendication 10, caractérisé en ce qu'il est présenté sous une forme administrable par voie orale.

12. Médicament conforme à la revendication 10, caractérisé en ce qu'il est présenté sous forme de solution injectable par voie intrapéritonéale.

**Revendications** pour l'Etat contractant: AT

1. Procédé de fabrication d'un sel de calcium ou de lithium acide de l'acide 2-oxo-1,5-pentanedioïque, caractérisé en ce que l'on fixe des ions de calcium ou de lithium sur une résine échangeuse d'ions à l'aide d'une solution d'un sel de calcium ou de lithium, puis on fait passer sur cette résine une solution aqueuse d'acide 2-oxo-1,5-pentanedioïque, on recueille l'effluent que l'on concentre jusqu'à cristallisation puis on filtre, lave et sèche les cristaux obtenus.

2. Procédé selon la revendication 1, caractérisé en ce que la résine échangeuse d'ions est une résine faiblement acide.

3. Procédé selon l'une des revendications 1 ou 2, caractérisé en ce que l'on fixe des ions de calcium ou de lithium sur la résine par percolation d'une solution d'hydroxyde, ou d'acétate de calcium ou de lithium.

4. Procédé selon l'une des revendications 1 ou 2, caractérisé en ce que l'on fixe des ions de calcium sur la résine par percolation d'une solution de chlorure de calcium après avoir fixé sur cette résine des ions de sodium ou d'ammonium.

5. Procédé selon l'une des revendications 1 à 4, caractérisé en ce que la résine échangeuse d'ions est une résine de type carboxylique.

6. Procédé selon l'une des revendications 1 à 5, caractérisé en ce que l'on concentre l'effluent aqueux de la résine sous pression réduite.

7. Procédé selon l'une des revendications 1 à 6, caractérisé en ce que l'on fait passer sur la résine une solution aqueuse, sensiblement normale, d'acide 2-oxo-1,5-pentanedioïque, puis de l'eau déminéralisée jusqu'à l'obtention d'un effluent neutre.

**Patentansprüche** für die Vertragsstaaten BE, CH, DE, GB, IT, LI, LU, NL, SE

1. Calciumsalz der 2-Oxopentan-1,5-disäure, gekennzeichnet durch die Formel:

$$(COOH-CH_2-CH_2-\underset{\underset{O}{\|}}{C}-COO)_2-Ca^{2+}, x\ H_2O$$

worin x eine ganze Zahl zwischen 0 und 4 ist.

2. Lithiumsalz der 2-Oxopentan-1,5-disäure, gekennzeichnet durch die Formel:

$$COOH-CH_2-CH_2-\underset{\underset{O}{\|}}{C}-COO^-, Li^+, H_2O$$

3. Verfahren zur Herstellung eines sauren Salzes gemäss einem der Ansprüche 1 oder 2, dadurch gekennzeichnet, dass man Calcium- oder Lithiumionen an einem Ionenaustauscherharz mit Hilfe einer Lösung eines Calcium- oder Lithiumsalzes fixiert, dann über dieses Harz eine wässerige Lösung der 2-Oxopentan-1,5-disäure laufen lässt, die abfliessende Lösung aufnimmt und bis zur Kristallisation konzentriert und anschliessend die erhaltenen Kristalle abfiltriert, wäscht und trocknet.

4. Verfahren nach Anspruch 3, dadurch gekennzeichnet, dass das Ionenaustauscherharz ein schwarch saures Harz ist.

5. Verfahren nach einem der Ansprüche 3 oder 4, dadurch gekennzeichnet, dass man Calcium- oder Lithiumionen auf dem Harz durch Perkolation mit einer Calcium- oder Lithiumhydroxid- oder -acetatlösung fixiert.

6. Verfahren nach einem der Ansprüche 3 oder 4, dadurch gekennzeichnet, dass man Calciumionen auf dem Harz durch Perkolation mit einer Calciumchloridlösung nach vorheriger Fixierung von Natrium- oder Ammoniumionen auf diesem Harz fixiert.

7. Verfahren nach einem der Ansprüche 3 bis 6, dadurch gekennzeichnet, dass das Ionenaustauscherharz ein carboxylgruppenhaltiges Harz ist.

8. Verfahren nach einem der Ansprüche 3 bis 7, dadurch gekennzeichnet, dass man die aus dem Harz abfliessende wässerige Reaktionslösung unter vermindertem Druck konzentriert.

9. Verfahren nach einem der Ansprüche 3 bis 8, dadurch gekennzeichnet, dass man über das Harz eine genau normale wässerige Lösung der 2-Oxopentan-1,5-disäure und dann entmineralisiertes Wasser bis zur Erzielung eines neutralen Abstroms laufen lässt.

10. Arzneimittel zur Behandlung krankhafter Zustände des Zentralnervensystems, dadurch gekennzeichnet, dass es als aktive Substanz ein Salz gemäss einem der Ansprüche 1 oder 2 enthält.

11. Arzneimittel nach Anspruch 10, dadurch gekennzeichnet, dass es in einer oral applizierbaren Darreichungsform vorliegt.

12. Arzneimittel nach Anspruch 10, dadurch gekennzeichnet, dass es in Form einer intraperitoneal injizierbaren Lösung vorliegt.

**Patentansprüche** für den Vertragsstaat: AT

1. Verfahren zur Herstellung eines sauren Calcium- oder Lithiumsalzes der 2-Oxopentan-1,5-disäure, dadurch gekennzeichnet, dass man Calcium- oder Lithiumionen an einem Ionenaustauscherharz mit Hilfe einer Lösung eines Calcium- oder Lithiumsalzes fixiert, dann über dieses Harz eine wässerige Lösung der 2-Oxopentan-1,5-disäure laufen lässt, die abfliessende Lösung aufnimmt und bis zur Kristallisation konzentriert und anschliessend die erhaltenen Kristalle abfiltriert, wäscht und trocknet.

2. Verfahren nach Anspruch 1, dadurch gekennzeichnet, dass das Ionenaustauscherharz ein schwach saures Harz ist.

3. Verfahren nach einem der Ansprüche 1 oder 2, dadurch gekennzeichnet, dass man Calcium- oder Lithiumionen auf dem Harz durch Perkolation mit einer Calcium- oder Lithiumhydroxid- oder -acetatlösung fixiert.

4. Verfahren nach einem der Ansprüche 1 oder 2, dadurch gekennzeichnet, dass man Calciumionen auf dem Harz durch Perkolation mit einer Calciumchloridlösung nach vorheriger Fixierung von Natrium- oder Ammoniumionen auf diesem Harz fixiert.

5. Verfahren nach einem der Ansprüche 1 bis 4, dadurch gekennzeichnet, dass das Ionenaustauscherharz ein carboxylgruppenhaltiges Harz ist.

6. Verfahren nach einem der Ansprüche 1 bis 5, dadurch gekennzeichnet, dass man die aus dem Harz abfliessende wässerige Reaktionslösung unter vermindertem Druck konzentriert.

7. Verfahren nach einem der Ansprüche 1 bis 6, dadurch gekennzeichnet, dass man über das Harz eine genau normale wässerige Lösung der 2-Oxopentan-1,5-disäure und dann entmineralisiertes Wasser bis zur Erzielung eines neutralen Abstroms laufen lässt.

**Claims** for the Contracting States BE, CH, DE, GB, IT, LI, LU, NL, SE

1. Calcium salt of 2-oxo-1,5-pentanedioic acid, characterized in that it has the formula:

$$(COOH-CH_2-CH_2-\underset{\underset{O}{\|}}{C}-COO)_2-Ca^{2+}, x\ H_2O$$

where x is a whole number comprised between 0 and 4.

2. Lithium salt of 2-oxo-1,5-pentanedioic acid, characterized in that it has the formula:

$$COOH-CH_2-CH_2-\underset{\underset{O}{\|}}{C}-COO^-\ Li^+, H_2O$$

3. Method of manufacture of an acid salt in accordance with either of Claims 1 or 2, characterized in that calcium or lithium ions are adsorbed on an ion-exchange resin by means of a solution of a calcium or lithium salt, whereupon an aqueous solution of 2-oxo-1,5-pentanedioic acid is passed through this resin, the effluent is collected and concentrated until crystallization, whereupon the crystals obtained are filtered, washed and dried.

4. Method in accordance with Claim 3, characterized in that the ion-exchange resin is a weakly acidic resin.

5. Method in accordance with either of Claims 3 or 4, characterized in that calcium or lithium ions are adsorbed on the resin by percolation of a solution of calcium or lithium hydroxide or acetate.

6. Method in accordance with either of Claims 3 or 4, characterized in that calcium ions are adsorbed on the resin by percolation of a calcium chloride solution after sodium or ammonium ions have been adsorbed on this resin.

7. Method in accordance with any of Claims 3 to 6, characterized in that the ion-exchange resin is a resin of carboxylic type.

8. Method in accordance with any of Claims 3 to 7, characterized in that the aqueous effluent of the resin is concentrated at low pressure.

9. Method in accordance with any of Claims 3 to 8, characterized in that a substantially normal aqueous solution of 2-oxo-1,5-pentanedioic acid followed by demineralized water is passed through the resin until a neutral effluent is obtained.

10. Medicament for the treatment of disorders of the central nervous system, characterized in that it contains as an active substance a salt in accordance with either of Claims 1 or 2.

11. Medicament in accordance with Claim 10, characterized in that it is presented in a form which can be administered by mouth.

12. Medicament in accordance with Claim 10, characterized in that it is presented in the form of a solution which can be administered by intraperitoneal injection.

**Claims** for the Contracting State: AT

1. Method of manufacture of an acid calcium or lithium salt of 2-oxo-1,5-pentanedioic acid, characterized in that calcium or lithium ions are adsorbed on an ion-exchanges resin by means of a solution of a calcium or lithium salt, whereupon an aqueous solution of 2-oxo-1,5-pentanedioic acid is passed through this resin, the effluent is collected and concentrated until crystallization, whereupon the crystals obtained are filtered, washed and dried.

2. Method in accordance with Claim 1, characterized in that the ion-exchange resin is a weakly acidic resin.

3. Method in accordance with either of Claims 1 or 2, characterized in that calcium or lithium ions are adsorbed on the resin by percolation of a solution of calcium or lithium hydroxide or acetate.

4. Method in accordance with either of Claims 1 or 2, characterized in that calcium ions are adsorbed on the resin by percolation of a calcium chloride solution after sodium or ammonium ions have been adsorbed on this resin.

5. Method in accordance with any of Claims 1 to 4, characterized in that the ion-exchange resin is a resin of the carboxylic type.

6. Method in accordance with any of Claims 1 to 5, characterized in that the aqueous effluent of the resin is concentrated at low pressure.

7. Method in accordance with any of Claims 1 to 6, characterized in that a substantially normal aqueous solution of 2-oxo-1,5-pentanedioic acid followed by demineralized water is passed through the resin until a neutral effluent is obtained.